# EUROPEAN PATENT APPLICATION

(11) **EP 1 958 615 A1**
(43) Date of publication of application: **20.08.2008**
(21) Application number: 07090018.8
(22) Date of filing: 13.02.2007
(51) Int. Cl.: A61K 9/14, A61K 9/16

(54) **Solid pharmaceutical formulations of a homogeneous dispersion of active principles having pH-dependent solubility**

(71) Applicant: Bayer Schering Pharma Aktiengesellschaft, 13353 Berlin (DE)
(72) Inventor: Juergens, Kai., 13505 Berlin (DE); Gysler, Jens., 14059 Berlin (DE)

(57) **Abstract**

The present invention relates to solid pharmaceutical formulations of a homogeneous dispersion of at least one active principle which possesses pH-dependent solubility, e.g. 1-[4-chloroanilino]-4-[4-pyridylmethyl]phthalazine, or a pharmaceutically acceptable salt thereof, to solid dosage forms comprising said solid pharmaceutical formulations, to methods of preparing said solid pharmaceutical formulations and said solid dosage forms, to uses of said solid pharmaceutical formulations, alone or in combination with one or more pharmaceutically active compounds, for the manufacture of a medicament for the treatment especially of a proliferative disease, such as cancer, and to a method of treatment of a proliferative disease, such as cancer.

## Description

### FIELD OF THE PRESENT INVENTION

The present invention relates to solid pharmaceutical formulations of a homogeneous dispersion of at least one active principle which possesses pH-dependent solubility, *e.g*. 1-[4-chloroanilino]-4-[4-pyridylmethyl]-phthalazine, or a pharmaceutically acceptable salt thereof, to solid dosage forms comprising said solid pharmaceutical formulations, to methods of preparing said solid pharmaceutical formulations and said solid dosage forms, to uses of said solid pharmaceutical formulations, alone or in combination with one or more pharmaceutically active compounds, for the manufacture of a medicament for the treatment especially of a proliferative disease, such as cancer, and to a method of treatment of a proliferative disease, such as cancer.

### BACKGROUND OF THE PRESENT INVENTION

It is widely known that drug substances with a good solubility in acidic environment and poor solubility at neutral and/or basic pH values often show a reduced bioavailability in patients with reduced stomach acidity, e.g. in patients suffering from achlorhydria. This reduced bioavailability is due to the fact that the drug substances are not dissolved completely when passing the pylorus. The maintenance of such a drug substance in solution throughout the gastro-intestinal tract (the "GIT"), is almost impossible due to the poor solubility of the drug substance. In general, for patients with unknown stomach pH for example, this will result in a high inter- and intra-individual fluctuation of the bioavailability (or "BA"). The situation is even worse for treatment regimes that involve a co-medication of stomach pH-modifying drugs such as common proton pump inhibitors (or "PPIs", *e.g*. omeprazol) or H₂-receptor blockers (*e.g*. ranitidine). These regimes are well known in chemotherapeutical therapies. If the main drug to be administered is of pH-dependent solubility, a therapy failure may be induced.

Additionally, it is important that the rate of dissolution of such a drug of pH-dependent solubility is sufficient that the complete drug is dissolved before the stomach content is transferred through the pylorus into the duodenum. Hence, an immediate release of the drug substance from the oral dosage form and the immediate dissolution in the stomach liquid is essentially required.

Strategies are therefore needed in order to overcome the insolubility of such a drug of pH-dependent solubility in the stomach and hence to reduce the BA fluctuations.

A common approach is to introduce an acid together with the drug substance. This is well-known for Sempera^{®} 7 tablets (which contain Itraconazole), for example, where the label recommends taking the tablet together with a Cola drink. It is due to the phosphoric acid in the Cola drink that the solubility of the Itraconazole is increased and that the BA is thus higher. However, the co-administration of a Cola drink may lead to further complications, as the patient compliance on this administration is not controllable. Additionally, the rate of dissolution of the drug in question is dependent upon the particle size and structure of the drug substance, it being possible that said rate be too slow to lead to an immediate dissolution, thus leading to the drug in question being leaked out through the pylorus, without being dissolved.

Hence other solutions to the above-mentioned problems are needed.

Now, taking a particular example, Patent Application WO 98/35958 describes generically a series of phthalazine derivatives with angiogenesis inhibiting activity and specifically 1-[4-chloroanilino]-4-[4-pyridylmethyl]phthalazine, including salts thereof, in particular the succinic acid salt thereof, as an interesting candidate for treatment of proloferative diseases, such as cancer. This compound is an angiogenesis inhibiting compound by virtue of it being an inhibitor of all three Vascular Endothelial Growth Factor ("VEGFR") kinases : the three VEGFR receptors have different affinities to the ligands. VEGF-A binds to VEGFR1 and VEGFR2 ; VEGF-B and Placental Growth Factor bind to VEGFR1 ; VEGF-A and processed forms of VEGF-C and VEGF-D bind to VEGFR-2 ; VEGF-C and VEGF-D bind to VEGFR-3.

VEGFR-3 is especially important for the growth of lymphatic vessels which play a role in tumour and metastases formation. Also, in other disease states, asthma, the lymphatic tissue is of major importance as it does remain in the alveoli after an acute inflammation and does not resolve like the blood vessels. This leads to the continuing susceptibility to stimulation by foreign agents.

All these receptors are transmembrane proteins. The signal of the VEGF variants is transmitted via the dimerisation of two receptor molecules inducing thereby an activation of the enzymatic activity at the intracellular C-terminal end. The enzymatic activity is a signal kinase, which transfers phosphates from ATP to itself (autophosphorylation) and to downstream signal molecules. This allows for interaction with an entire intracellular signal cascade eventually leading to endothelial cell proliferation and migration. The macroscopic result is the formation of new vessels.

The inhibition is not dependent on a specific ligand, but blocks all the signals.

### 1-[4-chloroanilino]-4-[4-pyridylmethyl]phthalazine has the chemical structure below :

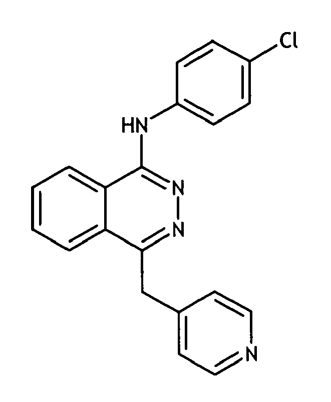

1-[4-chloroanilino]-4-[4-pyridylmethyl]phthalazine is also known as (4-chlorophenyl)[4-(4-pyridylmethyl)-phthalazin-1-yl], or ZK 226343, and is hereinafter referred to under the generic term of "PTK/ZK base". The succinate salt of 1-[4-chloroanilino]-4-[4-pyridylmethyl]phthalazine is known as PTK/ZK, PTK 787, CGP-79787D, ZK 222584, or Vatalanib succinate, and is hereinafter referred to under any of these terms, in particular "Vatalanib succinate", "the drug", or "the drug substance".

The solubility of Vatalanib succinate is extremely dependent on pH. Vatalanib succinate has a reasonable solubility at very low pH values, whereas the solubility decreases significantly as the pH is increased :

| pH | Buffered | Solubility | (mg/ml) |
|---|---|---|---|
| | | 37°C | 20°C |
| 1.0 | no | 108 | |
| 1.1 | yes | | 83 |
| 2.0 | no | 146 | |
| 3.0 | yes | 7.9 | |
| 3.1 | yes | | 7.2 |
| 3.6 | no | 0.35 | |
| 3.7 | no | | 0.34 |
| 4.5 | yes | 0.02 | |
| 5.0 | yes | 3.7 x 10⁻³ | 2.9 x 10⁻³ |
| 7.0 | yes | 7.1 x 10⁻⁴ | 3.1 x 10⁻⁴ |

Since Vatalanib succinate is absorbed in the small intestine, where the pH is usually above 5, it is of utmost importance that essentially all of the Vatalanib succinate is dissolved before entering the small intestine, i.e. essentially all of the Vatalanib succinate should be dissolved in the gastric juice, *i.e.* in the stomach. Clearly, in order to obtain satisfactory absorption of the Vatalanib succinate, it must be administered in an immediate-release formulation. More particularly, since a dosage form is taken orally, the form enters the stomach, wherein, normally, the pH is physiologically below 3.5. However, due to any given reason, such as co-medication with a proton pump inhibitor, or an H₂ receptor blocker, or with another stomach-pH-modifying compound, or due to a pathological stomach disorder, for example, there is a significant percentage of the human population that has a pH of the stomach of equal to or greater than 3.5. Said patients show significantly reduced bioavailability of the drug. Hence, the variability of the bioavailability of the drug within any given patient community is high. There is thus a risk of non-response to, or overdose of, said drug. Hence, there is a long-felt need, for the patient community as a whole, for a formulation which reduces the impact of the stomach pH on the bioavailability of the drug.

Very surprisingly, these important technical problems have been unexpectedly solved by the present inventors upon the discovery that a solid pharmaceutical formulation of a homogeneous dispersion of Vatalanib succinate and a pharmaceutically acceptable carboxylic acid, as prepared by an extrusion process, a melt process, particularly a melt-extrusion process, surprisingly provides an immediate-release and high-load solid pharmaceutical formulation comprising 1-[4-chloroanilino]-4-[4-pyridylmethyl]phthalazine, or a pharmaceutically acceptable salt thereof, whose homogeneous dispersion possesses an extremely high degree of dispersity (or even a "solid solution"), which enables the drug to be dissolved even in media of otherwise non-dissolving pH, particularly in the stomach for example, and which is thus very convenient for the patient in that the stomach pH is reduced thus enabling an enhanced dissolution of the drug, and thus an increased bioavailability thereof. Said solid pharmaceutical formulation of a homogeneous dispersion of Vatalanib succinate and a pharmaceutically acceptable carboxylic acid may be obtained, but not exclusively, by melting, extruding, or melt extruding, the carboxylic acid and Vatalanib succinate. The patient can therefore take the drug orally without the pH of the patient's stomach having to be taken into account. The formulation of the present invention provides the further surprising technical advantage in that the acidity of the formulation is not harmful to the stomach. More surprisingly, it was found that the formulations of the present invention reduce the variability of the bioavailable amount of Vatalanib succinate when administered orally. Particularly surprisingly, it was found that the formulations of the present invention can be stored under ambient conditions over long periods of time. This added technical advantage thus makes said formulation indeed suitable for clinical supply and commercialisation. More particularly surprisingly, it was found that when the solid pharmaceutical formulations of the present invention contains Vatalanib succinate, *i.e.* in "salified" form, the drug substance does not undergo any trans-salification phenomena.

Thus, the object of the present invention is to provide a solid pharmaceutical formulation of a homogeneous dispersion of at least one active principle which possesses pH-dependent solubility, *e.g*. 1-[4-chloroanilino]-4-[4-pyridylmethyl]phthalazine, or a pharmaceutically acceptable salt thereof, which provides a solution to the technical problems mentioned above. This object is met by said solid pharmaceutical formulations described in the present text and defined in the claims appended hereto.

### SUMMARY OF THE PRESENT INVENTION

In a first aspect, the present invention provides a solid pharmaceutical formulation of a homogeneous dispersion of :
a) a pharmaceutically acceptable salt of 1-[4-chloroanilino]-4-[4-pyridylmethyl]phthalazine ; and
b) a pharmaceutically acceptable carboxylic acid having a pKa value of equal to or less than 3.2, as coherent phase ;
in which at least 70% of said 1-[4-chloroanilino]-4-[4-pyridylmethyl]phthalazine, or pharmaceutical acceptable salt thereof, is released from said solid pharmaceutical formulation within 30 minutes as determined by the USP XXVI paddle method or rotating paddle method using non-buffered aqueous media of pH 6.8 at 37°C as the dissolution media and 100 rpm stirring rate.

In a second aspect, the present invention provides a solid pharmaceutical formulation as described above, which is in the form of a tablet, a pellet or a powder.

In a third aspect, the present invention provides a method of preparing a solid pharmaceutical formulation of a homogeneous dispersion of a) a pharmaceutically acceptable salt of 1-[4-chloroanilino]-4-[4-pyridylmethyl]phthalazine, and b) a pharmaceutically acceptable carboxylic acid having a pKa value of equal to or less than 3.2, as coherent phase, said method comprising the steps of :
i) blending a mixture of said 1-[4-chloroanilino]-4-[4-pyridylmethyl]phthalazine, or a pharmaceutically acceptable salt thereof, and said pharmaceutically acceptable carboxylic acid having a pKa value of equal to or less than 3.2, thereby forming a blended mixture ;
ii) extruding or melting, preferably melt-extruding, and optionally spheronising, said blended mixture, thereby forming an extrudate or melt, particularly in a pellet form ;
iii) obtaining an appropriate particle size distribution of said extrudate or melt, for example by milling or sieving, thereby providing a extrudate or melt of appropriate particle size, referred to as the "solid pharmaceutical formulation", of the present invention.

In a fourth aspect, the present invention provides a solid pharmaceutical formulation obtainable by the method as described *supra.*

In a fifth aspect, the present invention provides a use of the solid pharmaceutical formulation as described *supra* as a medicament.

In a sixth aspect, the present invention provides a use of the solid pharmaceutical formulation as described *supra,* for the manufacture of a medicament for the treatment of a proliferative disease, particularly cancer.

In a seventh aspect, the present invention provides a method of treating a proliferative disease, particularly cancer, said method comprising administering a therapeutically effective amount of the solid pharmaceutical formulation as described *supra,* to a patient in need thereof.

Further aspects of the present invention, and embodiments thereof, will be apparent from the description *infra* and the appended claims.

### BRIEF DESCRIPTION OF THE DRAWINGS

The invention is better understood, and other objects, features and advantages of the present invention are clearly shown, from the following description which refers to the appended Figures. These are given solely as non-limiting examples which illustrate the invention.
Figure 1 shows the dissolution profiles of tablets manufactured according to Example 3, *i.e*. tablets containing a physical mixture of Vatalanib succinate and citric acid, as prepared by blending according to Example 2, after one month of storage at ambient conditions wherein :
   -◆-Start
   -■-1 Month 25°C/60% rel.hum.
Figure 2 shows the dissolution profiles of tablets manufactured according to Example 4, *i.e.* tablets containing a melt extrusion of Vatalanib succinate and citric acid, as prepared by melt extrusion according to Example 1, after one and three months of storage at ambient conditions wherein :
   -●- Start
   -◆- 1 Month 25°C/60% rel. hum.
   -■- 3 Months 25°C/60% rel. hum.

### DETAILED DESCRIPTION OF THE PRESENT INVENTION

As mentioned *supra,* in a first aspect, the present invention provides a solid pharmaceutical formulation of a homogeneous dispersion of :
a) a pharmaceutically acceptable salt of 1-[4-chloroanilino]-4-[4-pyridylmethyl]phthalazine ; and
b) a pharmaceutically acceptable carboxylic acid having a pKa value of equal to or less than 3.2, as coherent phase ;
in which at least 70% of said 1-[4-chloroanilino]-4-[4-pyridylmethyl]phthalazine, or pharmaceutical acceptable salt thereof, is released from said solid pharmaceutical formulation within 30 minutes as determined by the USP XXVI paddle method or rotating paddle method using non-buffered aqueous media of pH 6.8 at 37°C as the dissolution media and 100 rpm stirring rate.

In a preferred embodiment, the present invention provides a solid pharmaceutical formulation as mentioned above, wherein at least 75%, preferably at least 80%, of said 1-[4-chloroanilino]-4-[4-pyridylmethyl]phthalazine, or pharmaceutically acceptable salt thereof, is released from said solid pharmaceutical formulation within 30 minutes.

In an embodiment of said formulation, said 1-[4-chloroanilino]-4-[4-pyridylmethyl]phthalazine is in the form of a pharmaceutically acceptable salt, preferably a pharmaceutically acceptable acid addition salt, particularly a succinate.

In an embodiment of said formulation, said pharmaceutically acceptable carboxylic acid having a pKa value of equal to or less than 3.2, is a mono-, di, tri-, or polycarboxylic acid. Preferably said mono-, di, tri-, or polycarboxylic acid is selected from the group consisting of : oxalic acid, glycerophosphoric acid, aspartic acid, particularly L-aspartic acid, malic acid, maleic acid, phosphoric acid, glutamic acid, alginic acid, pamoic acid, 2-oxo-glutaric acid, malonic acid, salicylic acid, tartaric acid, particularly (+)-L-tartaric acid, fumaric acid, galactaric acid, citric acid, D-glucuronic acid, lactobionic acid. It is understood that said acid can exist in an anhydrous or hydrated form, particularly as a mono- or di-hydrate.

Particularly, said acid is citric acid or fumaric acid.

Said acids mentioned *supra* can exist in an anhydrous, mono-, di-, or other hydrated form.

In an embodiment, the present invention provides a solid pharmaceutical formulation as mentioned above, wherein at least one pharmaceutically-acceptable excipient is additionally present.

In another embodiment of said formulation, said pharmaceutically-acceptable excipient is a filler (d), preferably a saccharide-containing filler, more preferably, said saccharide-containing filler is selected from the group comprising, preferably consisting of, lactose, maltodextrin, sucrose, and mannitol. Preferably, said saccharide-containing filler is mannitol.

As will also be understood from the present disclosure, it is of utmost importance that the mixing ratio of the (a) drug substance and (b) carboxylic acid is set in such a way that a mixture at the given dose yields a decrease of the pH value of the non-buffered dissolution media sufficient to enable a dissolution of the drug substance.

In an embodiment, in said formulation, the ratio of component a) : component b) is 0.1 : 10 (*i.e.* any sub-range comprised between 0.1 : 10, *e.g*. 1:10, 2:9, 3:8, 4:7, 5:6 etc.), preferably 1 : 5, more preferably 1 : 2, by weight. Particularly, the ratio of component a) : component b) is 1 : 1, by weight.

In accordance with an embodiment, said solid pharmaceutical formulation contains said filler (d) in an amount of around 0 to 45%, preferably around 35 to 40%, more preferably around 36.9%, of the total weight of said solid pharmaceutical formulation.

As mentioned *supra,* in accordance with a second aspect, the present invention provides a solid pharmaceutical formulation as described above, which is in the form of a tablet, a pellet or a powder.

In an embodiment, in said tablet, said 1-[4-chloroanilino]-4-[4-pyridylmethyl]phthalazine, or pharmaceutically acceptable salt thereof, is present in an amount of around 15 to 50%, preferably around 25 to 30%, more preferably 26.8%, by weight of the total weight of tablet.

Particularly, said tablet is coated, particularly film-coated.

In an embodiment, said tablet is a sugar-coated.

In an embodiment, the above-mentioned powder is present in a capsule, particularly a hard gelatin capsule. Preferably, said capsule is coated, preferably film-coated.

In an embodiment, said powder is present in a single dose container, particularly a sachet, more particularly a stick pack.

As mentioned *supra,* in accordance with a third aspect, the present invention provides a method of preparing a solid pharmaceutical formulation of a homogeneous dispersion of a) a pharmaceutically acceptable salt of 1-[4-chloroanilino]-4-[4-pyridylmethyl]phthalazine, and b) a pharmaceutically acceptable carboxylic acid having a pKa value of equal to or less than 3.2, as coherent phase, said method comprising the steps of :
i) blending a mixture of said 1-[4-chloroanilino]-4-[4-pyridylmethyl]phthalazine, or a pharmaceutically acceptable salt thereof, and said pharmaceutically acceptable carboxylic acid having a pKa value of equal to or less than 3.2, thereby forming a blended mixture ;
ii) extruding or melting, preferably melt-extruding, and optionally spheronising, said blended mixture, thereby forming an extrudate or melt, particularly in a pellet form ;
iii) obtaining an appropriate particle size distribution of said extrudate or melt, for example by milling or sieving, thereby providing a extrudate or melt of appropriate particle size, which is referred to as the "solid pharmaceutical formulation" of the present invention.

In an optional embodiment, said method further comprises the step of:
iv) blending said extrudate of appropriate particle size with at least one pharmaceutically acceptable excipient, and optionally forming, particularly by direct compression, a tablet.

Optionally, said method further comprises the step of :
v) placing said extrudate into a capsule, preferable a hard gelatine capsule.

Optionally, said method further comprises the step of :
vi) placing said extrudate into a single dose container, preferably a sachet.

As mentioned *supra,* in accordance with a fourth aspect, the present invention provides a solid pharmaceutical formulation obtainable by the method as described *supra.*

As mentioned *supra,* in accordance with a fifth aspect, the present invention provides a use of the solid pharmaceutical formulation as described *supra* as a medicament.

As mentioned *supra,* in accordance with a sixth aspect, the present invention provides a use of the solid pharmaceutical formulation as described *supra,* for the manufacture of a medicament for the treatment of a proliferative disease, particularly cancer.

In an embodiment, said use can be in combination with a chemotherapeutic agent.

As mentioned *supra,* in accordance with a seventh aspect, the present invention provides a method of treating a proliferative disease, particularly cancer, said method comprising administering a therapeutically effective amount of the solid pharmaceutical formulation as described *supra,* to a patient in need thereof.

In an embodiment, said method comprises administering a therapeutically effective amount of the solid pharmaceutical formulation as described *supra,* in combination with a chemotherapeutic agent, to a patient in need thereof.

It is to be understood that the present invention covers all possible combination: of the embodiments described herein.

### In re. component a) : 1-[4-chloroanilino]-4-[4-pyridylmethyl]phthalazine :

1-[4-chloroanilino]-4-[4-pyridylmethyl]phthalazine may be the free base, or, indeed, a pharmaceutically acceptable salt thereof, in particular an acid addition salt. Such salts may be formed from suitable inorganic or organic acids. Examples of suitable inorganic acids include the halogen acids, such as hydrochloric acid ; sulphuric acid ; and phosphoric acid. Examples of suitable organic acids include phosphonic, sulphonic or sulphamic acids, carboxylic acids, such as acetic acid, propionic acid, octanoic acid, decanoic acid, dodecanoic acid, glycolic acid, lactic acid, 2-hydroxybutyric acid, gluconic acid, glucose monocarboxylic acid, fumaric acid, succinic acid, adipic acid, pimelic acid, suberic acid, azelaic acid, malic acid, tartaric acid, citric acid, glucaric acid, galactaric acid ; amino acids, such as glutamic acid, aspartic acid, N-methylglycine, acetylaminoacetic acid, N-acetylasparagine and N-acetylcysteine ; pyruvic acid, acetoacetic acid, phosphoserine, 2- or 3-glycerophosphoric acid, glucose-6-phosphoric acid, glucose-1-phosphoric acid, fructose-1,6-bis-phosphoric acid, maleic acid, hydroxymaleic acid, methylmaleic acid, cyclohexanecarboxylic acid, adamantanecarboxylic acid, benzoic acid, salicylic acid, 1- or 3-hydroxynaphthyl-2-carboxylic acid, 3,4,5-trimethoxybenzoic acid, 2-phenoxybenzoic acid, 2-acetoxybenzoic acid, 4-aminosalicylic acid, phthalic acid, phenylacetic acid, mandelic acid, cinnamic acid, glucuronic acid, galacturonic acid, methane- or ethanesulphonic acid, 2-hydroxyethanesulphonic acid, ethane-1,2-disulphonic acid, 2-, 3-, or 4-methylbenzenesulphonic acid, methylsulphuric acid, ethylsulphuric acid, dodecylsulphuric acid, N-cyclohexylsulphamic acid, N-methyl-, N-ethyl-, or N-propyl-sulphamic acid, or other organic acids, such as ascorbic acid.

### In re. component b) : said pharmaceutically acceptable carboxylic acid having a pKa value of equal to or less than 3.2 :

Said acid can be selected from the list mentioned in the publication International Union of Pure and Applied Chemistry (IUPAC), "Handbook of Pharmaceutical Salts, Properties, Selection, and Use", P. Heinrich Stahl, Camille G. Wermuth (Eds.), Wiley-VCH page 334 to 337, Table 2, which is herein incorporated by reference in its entirety.

### In re. pharmaceutically-acceptable excipients :

### Filler (d) :

Notably, said filler (d) is known to the person skilled in the art and can be, for example :
- an inert diluent or filler, such as a saccharide-containing filler, such as sucrose, maltodextrin, sorbitol, a sugar, mannitol, particularly Pearlitol SD 200, micro-crystalline cellulose, a starch, such as corn starch, sodium chloride, sodium phosphate, calcium carbonate, calcium phosphate, calcium sulphate or lactose, *e.g.* lactose monohydrate.

### Additional pharmaceutically-acceptable excipients :

Notably, said additional pharmaceutically-acceptable excipients are known to the person skilled in the art and can be, for example :
- a flow agent, such as highly dispersed silicon dioxide. The flow agent is typically present in an amount from 0.1-10% by weight of the total formulation. Preferably, the lubricant is present in an amount from 0.2-5% by weight of the total formulation, such as from 0.3-5% by weight of the total formulation, *e.g.* from 0.3-1% by weight of the total formulation, more preferably in an amount about 0.5% by weight of the total formulation.
- a lubricant, including a glidant and an anti-adhesive, such as magnesium stearate, zinc stearate, stearic acid, silicas, hydrogenated vegetable oils or talc. The lubricant is typically present in an amount from 0.1-10% by weight of the total formulation. Preferably, the lubricant is present in an amount from 0.2-5% by weight of the total formulation, such as from 0.5-5% by weight of the total formulation, *e.g.* from 1-5% by weight of the total formulation, more preferably in an amount from 1-3% by weight of the total formulation. In a preferred embodiment of the present invention, the lubricant is magnesium stearate.
- a disintegrant, such as a carboxymethylcellulose-based disintegrant, such as croscarmellose sodium, such as Ac-Di-Sol (marketed by FMC BioPolymer), a cross-linked povidone, sodium starch glycolate, corn starch, which may be gelatinised or not, or potato starch.
- a binder, such as pre-gelatinised corn starch.
- a surfactant and a wetting agent, such as naturally occurring phosphatides, *e.g*. lechitin or soybean lechitin ; condensation products of ethylene oxide with *e.g.* a fatty acid, a long chain fatty alcohol, or a partial ester derived from fatty acids and a hexitol or a hexitol anhydride, for example polyoxyethylene stearate, polyoxyethylene sorbitol monooleate, polyoxyethylene sorbitan monooleate, etc. ; or salts of long-chain aliphatic phosphates, such as sodium lauryl sulphate.

Examples of other pharmaceutically acceptable excipients which may be incorporated in the solid pharmaceutical formulation of the present invention include colorants, flavouring agents, plasticizers, humectants, buffering agents, etc.

### Solid pharmaceutical formulation

As will be understood from the present application, including the Examples provided herein, it is of utmost importance that the drug is released in a fast and reliable manner under acidic conditions. Thus, in the present context, the terms "fast-release" or "immediate-release" mean that at least 70% of the drug, *e.g*. 1-[4-chloroanilino]-4-[4-pyridylmethyl]phthalazine, or a pharmaceutically acceptable salt thereof, is dissolved from the solid pharmaceutical formulation within 30 minutes as determined by the USP XXVI paddle method or rotating paddle method using non-buffered aqueous media of pH 6.8 at 37°C as the dissolution media and 100 rpm stirring rate.

As will also be understood from the present disclosure it is of utmost importance that the tablet in accordance with the present invention contains a "high-load" of drug due to the relative large amount of drug needed for administration to patients. Thus, in the present context, the terms "high-load" or "high-drug-load" mean that the tablet contains at least 15% by weight, particularly at least 20% by weight, at least 25% by weight, at least 30% by weight, at least 35% by weight, at least 40% by weight, at least 45% by weight, at least 50% by weight, at least 55% by weight, calculated on the basis of the total weight of the tablet, of the drug *e.g.* 1-[4-chloroanilino]-4-[4-pyridylmethyl]phthalazine or a pharmaceutically acceptable salt thereof. Stated otherwise, said tablet, as mentioned *supra,* contains 20 to 55% by weight, calculated on the basis of the total weight of the tablet, of the drug *e.g.* 1-[4-chloroanilino]-4-[4-pyridylmethyl]phthalazine or a pharmaceutically acceptable salt thereof.

As used herein, the term "coherent phase" is understood as meaning the outer phase of a dispersion, that encloses the inner phase completely and is continuous.

As used herein, the term "second phase" is understood as meaning the inner phase of at least one "outer" phase of a dispersion, that is bicontinuous or discontinuous in the coherent phase.

As used herein, the term "homogeneous dispersion" is understood as meaning that in the solid pharmaceutical formulation of the present invention, the elements of the second phase are positioned homogenously within the coherent phase. Within the context of the present invention, the term "homogeneous dispersion" is understood as meaning a solid pharmaceutical formulation which is prepared by extrustion, particularly melt-extrusion, for example.

The solid pharmaceutical formulations of the present invention may also contain further drug substances, such as anti-cancer agents.

In those cases where the pharmaceutical formulation is in the form of a solid dosage form, in particular a solid unit dosage form (e.g. a tablet, sachet, in particular a stick pack, or capsule, particularly a hard gelatin capsule, in particular a tablet), the unit dosage form is adapted for oral administration and may be provided with a coating, such as a film coating, a sugar coating, or the like. Dosage forms for drugs, such as those mentioned above, are well-known to the person skilled in the art of drug formulation.

Thus, a suitable coating for the solid unit dosage form according to the invention may, for example, be a sugar coating or a film coating based on one or more of the ingredients : hydroxypropyl methylcellulose (or "HPMC"), methylcellulose, ethylcellulose, hydroxyethyl methylcellulose, hydroxypropylcellulose, carboxymethylcellulose sodium, acrylate polymers (*e.g*. Eudragit^{®} (marketed by Degussa), polyethylene glycols or polyvinylpyrrolidone, or a colorant, such as talc, titanium dioxide, a ferrous pigment. Preferably, the coating is a film coating based on HPMC.

### Methods

As used herein, in the context of the method of preparing the solid pharmaceutical formulation of the present invention, (*i.e*. the solid pharmaceutical formulation of a homogeneous dispersion of a) a pharmaceutically acceptable salt of 1-[4-chloroanilino]-4-[4-pyridylmethyl]-phthalazine, and b) a pharmaceutically acceptable carboxylic acid having a pKa value of equal to or less than 3.2, as coherent phase), it is to be understood that the initial step of blending said mixture of said two constituents a) and b) (*i.e.* in the step preceding that of extrusion or melting, preferably melt-extrusion), is one that involves mixing or blending under dry conditions, *i.e*. without the addition of liquid, e.g. water, in order to circumvent the formation of the compound para-chloroaniline, which is formed as a decomposition product of 1-[4-chloroanilino]-4-[4-pyridylmethyl]phthalazine under aqueous and acidic conditions, since said para-chloroaniline is well-known to be carcinogenic.

As used herein, the term "extrusion", as used in the context of the method of preparation of the formulations of the present invention, is understood as meaning a manufacturing process which is used to process a material or a blend of more than one material through a defined orifice, thereby giving rise to a homogeneous dispersion which possesses an extremely high degree of dispersity (or even a "solid solution"). In particular, in pharmaceutical technology, extrusion comprises an optional blending of different materials if present and the processing of these through a dice, often including an increase in pressure. The term "melt extrusion" as used herein is understood as meaning a special form of extrusion, where the temperature of the extruded material is raised beyond the melting temperature at a given process pressure of at least one of the components of the processed material. As is well-known to the person skilled in the art, the higher the process pressure, the lower the melting temperature of the components is. Consequently, the pressure used in the method of the invention is as high as acceptably possible.

### Medical use

The present invention provides means and methods for treating certain cancers or tumours or tumour angiogenesis, in any suitable animal, preferably in a mammal, and in particular in a human being.

The term "solid malignant tumour" is intended to indicate an abnormal malignant mass of tissue that is not inflammatory, which arises without obvious cause from cells of preexistent tissue, which possesses no physiologic function and which has the ability to invade normal cells and spread throughout the body. Examples of typical solid malignant tumours include breast carcinoma, non-small cell lung cancer, colon carcinoma, renal cell carcinoma and malignant melanoma.

The term "carcinoma" is intended to indicate a malignant tumour of epithelial origin. Epithelial tissue covers or lines the body surfaces inside and outside the body. Examples of epithelial tissue are the skin and the mucosa and serosa that line the body cavities and internal organs, such as intestines, urinary bladder, uterus, etc. Epithelial tissue may also extend into deeper tissue layers to from glands, such as mucus-secreting glands.

The term "sarcoma" is intended to indicate a malignant tumour growing from connective tissue, such as cartilage, fat, muscles, tendons and bones.

The term "leukaemia" is intended to indicate a blood cancer, *i.e*. a cancer that originates from the bone marrow and which keeps the marrow from producing normal red and white blood cells and platelets.

The term "lymphoma" refers to a cancer that originates in the nodes or glands of the lymphatic system.

The term "glioma", when used herein, is intended to cover a malignant tumour originating from glial cells.

The term "inhibition" or "inhibit" as used in connection with treatment of solid tumours is intended to cover the delayed appearance of primary or secondary tumours, slowed development of primary or secondary tumours, decreased occurrence of primary or secondary tumours, slowed or decreased severity of secondary effects of disease, arrested tumour growth and regression of tumours. The term "reduction" or "reducing" in connection with tumour size is covered by the term "inhibition" or "inhibit". The reduction of the solid tumour refers to a reduction of the tumour volume. For example, a 10% reduction of a solid tumour means that the volume of the treated tumour has been reduced with 10%.

An important aspect of the present invention lies in the therapeutic application of the solid pharmaceutical formulation of the present invention.

Thus, the present invention is also directed to a solid pharmaceutical formulation of the present invention for use as a medicament. In particular, the present invention is directed to use of the solid pharmaceutical formulation of the present invention for the manufacture of a medicament for treatment of cancer. Alternatively stated, the present invention is directed to a method of treating cancer, said method comprising administering a therapeutically effective amount of the solid pharmaceutical formulation of the present invention to a patient in need thereof.

In the present context the term "treatment of a cancer" or "treating a mammal having a cancer" is intended to mean that the solid pharmaceutical formulation described herein is administered in a therapeutically effective amount which is sufficient to *i)* inhibit growth of the tumour, *ii)* facilitate tumour regression, *i.e*. reduce the size of the tumour, *iii)* remove the tumour and/or *iv)* inhibit cancer cell metastasis.

The solid pharmaceutical formulation will be administered to patients in a "therapeutically effective" dose, *i.e.* in a dose that is sufficient to produce the desired effects in relation to the condition for which it is administered. The exact dose will depend on the cancer form to be treated, and will be ascertainable by one skilled in the art using known techniques. A suitable dose of the drug, in particular Vatalanib succinate, is contemplated to be in the range of about 0.5-3 g/patient/day, preferably 1-2 g/patient/day, such as 1-1.5 g/patient/day, *e.g.* 1.3-1.4 g/patient/day, in particular 1.34 g/patient/day.

In a very interesting embodiment of the present invention said cancer is a carcinoma, such as a carcinoma selected from the group consisting of malignant melanoma, basal cell carcinoma, ovarian carcinoma, breast carcinoma, non-small cell lung cancer, renal cell carcinoma, bladder carcinoma, recurrent superficial bladder cancer, stomach carcinoma, prostatic carcinoma, pancreatic carcinoma, lung carcinoma, cervical carcinoma, cervical dysplasia, laryngeal papillomatosis, colon carcinoma, colorectal carcinoma and carcinoid tumours, in particular selected from the group consisting of malignant melanoma, non-small cell lung cancer, breast carcinoma, colon carcinoma and renal cell carcinoma.

Thus, in one embodiment of the present invention said cancer is malignant melanoma, such as superficial spreading melanoma, nodular melanoma, lentigo maligna melanoma, acral melagnoma, amelanotic melanoma or desmoplastic melanoma. In another embodiment of the present invention said cancer is non-small cell lung cancer. In still another embodiment of the present invention said cancer is breast carcinoma. In a further embodiment of the present invention said cancer is colon carcinoma. In an even further embodiment of the present invention said cancer is renal cell carcinoma.

In a further interesting embodiment of the present invention said cancer is a sarcoma, such as a sarcoma selected from the group consisting of osteosarcoma, Ewing's sarcoma, chondrosarcoma, malignant fibrous histiocytoma, fibrosarcoma and Kaposi's sarcoma.

In an even further interesting embodiment of the present invention said cancer is a glioma.

As will be understood by the skilled person, the above-mentioned cancer types (*i.e*. carcinomas, sarcomas and gliomas) are characterised by the presence of solid tumours.

In an even further interesting embodiment of the present invention said cancer is a lymphoma, such as a lymphoma selected from the group consisting of Hodgkin's disease, non-Hodgkin's disease, B-cell lymphoma, T-cell lymphoma, follicular lymphoma, Burkitt's lymphoma and mycosis fungoides.

In still another interesting embodiment of the present invention said cancer is a myeloma, such as multiple myeloma.

One important use in cancer therapy is the reduction and blockade of ascites formation in abdominal tumours of different origin (*e.g.* uterine endometrium, ovarial cancer, colon cancer) also for mesothelioma.

In addition to its use as anticancer agent, the formulation of the present invention can be used for other diseases characterised by overshooting angiogenesis, such as macular degeneration due to vessel in-growth, corneal transplatation due to lymphatic vessel ingrowth and thereby breakage of the immune privilege. It is helpful in diseases such as rheumatoid arthritis with vessels growing ectopically towards the joints. As mentioned above, it is effective in asthma. Also diseases with female cycle associated vessel growth are influenced beneficially as is the case for endometriosis.

It will be understood that an even more effective treatment of the various cancer forms may be obtained by combination therapy where the solid pharmaceutical formulation of the present invention is combined with a suitable chemotherapeutic agent and/or is combined with radiotherapy and/or is combined with surgery.

Thus, a further aspect the present invention relates to the use of a solid pharmaceutical formulation of the present invention for the manufacture of a medicament for treatment of cancer in combination with a chemotherapeutic agent. Analogously, a further aspect of the present invention relates to a method of treating cancer, said method comprising administering a therapeutically effective amount of the solid pharmaceutical formulation of the present invention and a chemotherapeutic agent to a patient in need thereof.

Specific examples of suitable chemotherapeutic agents include chemotherapeutic agents selected from the group consisting of adrenocorticosteroids, such as prednisone, dexamethasone or decadron ; altretamine (hexalen, hexamethylmelamine (HMM)) ; amifostine (ethyol) ; aminoglutethimide (cytadren) ; amsacrine (M-AMSA) ; anastrozole (arimidex) ; androgens, such as testosterone ; asparaginase (elspar) ; bacillus calmette-gurin ; bicalutamide (casodex) ; bleomycin (blenoxane) ; busulphan (myleran) ; carboplatin (paraplatin) ; carmustine (BCNU, BiCNU) ; chlorambucil (leukeran) ; chlorodeoxyadenosine (2-CDA, cladribine, leustatin) ; cisplatin (platinol) ; cytosine arabinoside (cytarabine) ; dacarbazine (DTIC) ; dactinomycin (actinomycin-D, cosmegen) ; daunorubicin (cerubidine) ; docetaxel (taxotere) ; doxorubicin (adriomycin) ; epirubicin ; estramustine (emcyt) ; estrogens, such as diethylstilbestrol (DES) ; etopside (VP-16, VePesid, etopophos) ; fludarabine (fludara) ; flutamide (eulexin) ; 5-FUDR (floxuridine) ; 5-fluorouracil (5-FU) ; gemcitabine (gemzar) ; goserelin (zodalex) ; herceptin (trastuzumab) ; hydroxyurea (hydrea) ; idarubicin (idamycin) ; ifosfamide ; IL-2 (proleukin, aldesleukin) ; interferon alpha (intron A, roferon A) ; irinotecan (camptosar) ; leuprolide (lupron) ; levamisole (ergamisole) ; lomustine (CCNU) ; mechlorathamine (mustargen, nitrogen mustard) ; melphalan (alkeran) ; mercaptopurine (purinethol, 6-MP) ; methotrexate (mexate) ; mitomycin-C (mutamucin) ; mitoxantrone (novantrone) ; octreotide (sandostatin) ; pentostatin (2-deoxycoformycin, nipent) ; plicamycin (mithramycin, mithracin) ; prorocarbazine (matulane) ; streptozocin ; tamoxifin (nolvadex) ; taxol (paclitaxel) ; teniposide (vumon, VM-26) ; thiotepa ; topotecan (hycamtin) ; tretinoin (vesanoid, all-trans retinoic acid) ; vinblastine (valban) ; vincristine (oncovin) and vinorelbine (navelbine).

Other chemotherapeutic agents, which may be useful for the purposes described herein include the chemotherapeutic agents mentioned in column 12, line 62 to column 13, line 42 of US 6,482,802. For a description of these and other chemotherapeutic agents, see The Merck Index, 12th edition, pp. THER 13-14. Both references being incorporated herein by reference in their entirety.

It will be understood that the chemotherapeutic agent is selected under due consideration of the actual cancer form. In a preferred embodiment of the present invention the following chemotherapeutic agents are used (together with the solid pharmaceutical formulation described herein) for treatment of the following specific cancer forms : malignant melanoma and cisplatin ; malignant melanoma and IL-2 ; renal cell carcinoma and doxorubicin ; renal cell carcinoma and IL-2 ; breast carcinoma and doxorubicin ; breast carcinoma and taxol ; colon carcinoma and 5-fluorouracil ; colon carcinoma and cisplatin ; and non-small cell lung cancer and cisplatin.

The invention is further described in the following Examples. The Examples should not, in any manner, be understood as limiting the generality of the present specification and claims.

### EXAMPLES

### EXAMPLE 1 : PREPARATION OF A MELT EXTRUSION OF VATALANIB SUCCINATE AND CITRIC ACID

Vatalanib succinate and critic acid monohydrate are given in equal parts (1000 gram each) into a blender, (a 10 litre Turbula-Mixer (Willy A. Bachofen GmbH, CH)) and mixed until a homogenous blend is achieved, for 15 minutes. The blend is filled into the loading funnel of a melt extrusion machine (Leistritz ZSE27 (Leistritz Extrusionstechnik GmbH, Nuremberg, Germany)). The blend is processed at a process temperature of approximately 55° to 65°C at low extrusion speed. The extruded mass is collected. After the complete cooling to ambient conditions, the extruded mass is screened through a 100 µm screen using a centrifugal mill, (Retsch ZM200 (Retsch GmbH, Haan, Germany)) under nitrogen atmosphere.

### EXAMPLE 2: PREPARATION OF A PHYSICAL MIXTURE OF VATALANIB SUCCINATE AND CITRIC ACID

Vatalanib succinate and citric acid monohydrate are given in equal parts (1000 gram each) into a blender, (a 10 litre Turbuta-Mixer (Willy A. Bachofen GmbH, CH)) and mixed until a homogenous blend is achieved, for 15 minutes.

### EXAMPLE 3 : PREPARATION OF IMMEDIATE RELEASE ACID TABLETS USING THE PHYSICAL MIXTURE OF VATALANIB SUCCINATE AND CITRIC ACID OF EXAMPLE 2.

Using the physical mixture of Vatalanib Succinate and citric acid monohydrate of Example 2, immediate release tablets are produced by firstly passing 53.6g of the mixture and 26.9g of mannitol as filler, (Pearlitol SD 200 (Roquette GmbH, Frankfurt, Germany)) and 15g pregeletanised corn starch as binder, (Starch 1500 (Colorcon, Idstein, Germany)) are given into an 250ml glass jar and blending it for 10min. at 36 rpm in a blender, (a Turbula mixer (Willy A. Bachofen, CH)). Then 2g croscarmellose as disintegrant, (Ac-Di-Sol (Lehmann & Voss, Hamburg, Germany)) is added, blended for 5 min. and finally 2.5g of magnesium stearate (WerbaChem, Vienna, Austria) is added and blended for additional 1 min.
The yielded press mass is given to a tablet press, such as a Korsch EK0 excenter press (Korsch GmbH, Berlin, Germany) equipped with oblong punch tools, such as Notter 13.75 x 5 curvature radius 4 (Notter GmbH, Ölbronn, Germany). During the compression the material showed strong sticking to the compression tools.

### EXAMPLE 4 : PREPARATION OF IMMEDIATE RELEAE TABLETS USING A MELT EXTRUDED VATALANIB SUCCINATE AND CITRIC ACID OF EXAMPLE 1.

Using the melt extruded mass of Example 1, immediate release tablets are produced by firstly passing 53.6g of the melt extruded mass and 0.5g of highly dispersed silicon dioxide, (Aerosil SD 200 (Degussa AG, Frankfurt, Germany)) through a sieve, (a 0.3 mm analytical sieve (Retsch GmbH, Haan, Germany)) into a 250 ml glass jar and blending it for 5min. at 36 rpm in a blender, (a Turbula mixer (Willy A. Bachofen, CH)). To this initial blend 36.9g of mannitol as filler, (Pearlitol SD 200 (Roquette GmbH, Frankfurt, Germany)) and 5g pregeletanised corn starch as binder, (Starch 1500 (Colorcon, Idstein, Germany)) are given and blended for 10min at 36 rpm. Then 2g croscarmellose as disintegrant, (Ac-Di-Sol (Lehmann & Voss, Hamburg, Germany)) is added, blended for 5 min. and finally 2g of magnesium Stearate (WerbaChem, Vienna, Austria) is added and blended for additional 1 min.
The yielded press mass is given to a tablet press, (a Korsch EK0 excenter press (Korsch GmbH, Berlin, Germany)) equipped with oblong punch tools, (Notter 13.75 x 5 curvature radius 4 (Notter GmbH, Ölbronn, Germany)).

Very surprisingly, the manufacturing of the tablets could be performed without any sticking of the press-mass to the compressing tools.

### EXAMPLES 5 TO 8 : PREPARATION OF PHYSICAL MIXTURES OF VATALANIB SUCCINATE AND ORGANIC ACIDS, AND PREPARATION OF IMMEDIATE RELEASE TABLETS OF SUCH MIXTURES.

In accordance with Example 2, physical mixtures of Vatalanib succinate and the following organic acids were prepared :

| **Example** | **Acid** |
|---|---|
| 5 | malic acid |
| 6 | maleic acid |
| 7 | tartaric acid |
| 8 | fumaric acid |

In accordance with Example 3, the yielded blends were used to prepare immediate release tablets. During the compression process, all press-masses show strong sticking of the material to the compression tools.

### EXAMPLE 9 : STORAGE OF ACIDIC VATALANIB SUCCINATE TABLETS OF EXAMPLE 3, OVER ONE MONTH.

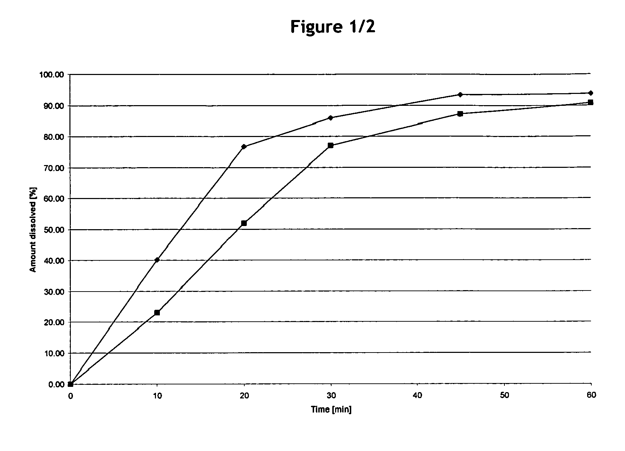

Uncoated tablets according to Examples 3 were examined for stability at 25°C / 60% relative humidity (RH) according to the International Committee of Harmonisation (ICH) Guidelines on Stability Studies. After one month of storage, the tablets were inspected visually. The tablets showed uneven surfaces with many cracks and openings. The integrity of the tablet was destroyed.
The release of the drug substance from the tablet was reduced compared to the start of the study as shown in Figure 1.

### EXAMPLE 10 : DETERMINATION OF VATALANIB SUCCINATE DISSOLUTION FROM TABLETS

Determination of Vatalanib succinate dissolution from tablets was performed in accordance with the USP XXVI Paddle Method using the following test parameters :

| | |
|---|---|
| Apparatus: | USP dissolution apparatus 2 with six covered glass vessels |
| | and paddle stirrers |
| Medium: | purified water, degassed. The pH value was finally adjusted |
| | to 6.8±0.05 |
| Filing volume: | 1000 ml |
| Temperature: | 37°C±0.5°C |
| Stirring rate: | 100 rpm±2 rpm |
| Sampling times: | 5, 10, 15, 30, 45 and 60 minutes |
| Detection: | UV-measurement at 316 nm |

An X-Ray diffractometrical analysis showed the formation of a novel phase with hitherto unknown structure.

### EXAMPLE 11 : STORAGE OF ACIDIC VATALANIB SUCCINATE TABLETS OF EXAMPLE 4, OVER THREE MONTHS.

Uncoated acidic tablets prepared according to Example 4 were examined for stability at 25°C / 60% relative humidity (RH) according to the International Committee of Harmonisation (ICH) Guidelines on Stability Studies. Surprisingly, it was found that the tablets show complete integrity and an almost unharmed surface.

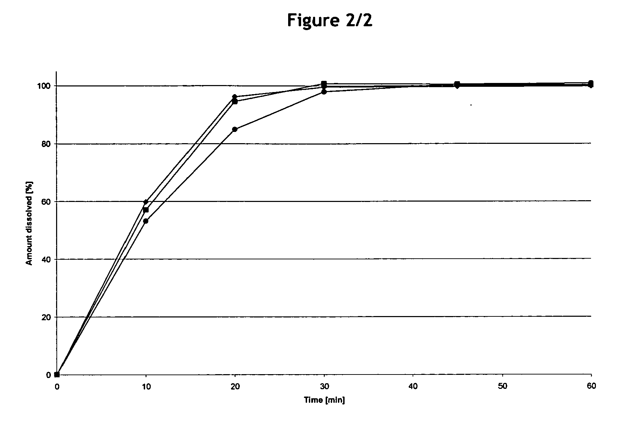

After one month of storage, and after three months of storage, the drug release was investigated in non-buffer water of pH 6.8, and the results are shown in Figure 2.

Surprisingly, it was found that the formulation showed no reduction of the dissolution rate over the storage time.

Additionally, the X-Ray diffractometrical analysis, does not reveal the formation of any additional phase and hence support the stability of the formulation.

In view of the *in vitro* dissolution profiles being unchanged upon storage, it is clear that this formulation is indeed suitable for clinical supply and commercialisation.

The *in vitro* dissolution profiles were acquired by using the method as described in Example 9.

As can be seen from Figure 2, (which refers to the tablets of Example 4, which contain a melt extrusion of Vatalanib succinate and citric acid, as prepared by melt extrusion according to Example 1), in comparison to Figure 1, (which refers to tablets of Example 3, which contain a physical mixture of Vatalanib succinate and citric acid, as prepared by physical blending (mixing) according to Example 2),very surprisingly :
i) the tablets of Example 4 displayed not only a faster rate of dissolution than those of Example 3 ; but
ii) also, the tablets of Example 4 actually displayed an acceptable increase in the rate of dissolution with storage time, *i.e.* the rate of dissolution increased after one month and after 3 months of storage, at ambient conditions, with respect to the start, *i.e*. before storage, it being more surprisingly noted that in Figure 1, it can be seen that the tablets of Example 3 actually displayed a decrease in the rate of dissolution with storage time.

## Claims

1. A solid pharmaceutical formulation, **characterised in that** it is of a homogeneous dispersion of :
a) a pharmaceutically acceptable salt of 1-[4-chloroanilino]-4-[4-pyridylmethyl]phthalazine; and
b) a pharmaceutically acceptable carboxylic acid having a pKa value of equal to or less than 3.2, as coherent phase ;
in which at least 70% of said 1-[4-chloroanilino]-4-[4-pyridylmethyl]phthalazine, or pharmaceutical acceptable salt thereof, is released from said solid pharmaceutical formulation within 30 minutes as determined by the USP XXVI paddle method or rotating paddle method using non-buffered aqueous media of pH 6.8 at 37°C as the dissolution media and 100 rpm stirring rate.

2. The formulation according to claim 1, wherein at least 75%, preferably at least 80%, of said 1-[4-chloroanilino]-4-[4-pyridylmethyl]phthalazine, or pharmaceutically acceptable salt thereof, is released from said solid pharmaceutical formulation within 30 minutes.

3. The formulation according to claim 1 or 2, wherein at least one pharmaceutically-acceptable excipient is additionally present.

4. The formulation according to any one of claims 1 to 3, wherein said 1-[4-chloroanilino]-4-[4-pyridylmethyl]phthalazine is in the form of a pharmaceutically acceptable salt thereof.

5. The formulation according to any one of claims 1 to 4, wherein said pharmaceutically acceptable salt thereof is an acid addition salt.

6. The formulation according to claim 5, wherein said pharmaceutically acceptable salt of 1-[4-chloroanilino]-4-[4-pyridylmethyl]phthalazine is a succinate.

7. The formulation according to any one of claims 1 to 6, wherein said pharmaceutically acceptable carboxylic acid having a pKa value of equal to or less than 3.2, is a mono-, di, tri-, or polycarboxylic acid.

8. The formulation according to claim 7, wherein said mono-, di, tri-, or polycarboxylic acid is selected from the group consisting of : oxalic acid, glycerophosphoric acid, aspartic acid, particularly L-aspartic acid, malic acid, maleic acid, phosphoric acid, glutamic acid, alginic acid, pamoic acid, 2-oxo-glutaric acid, malonic acid, salicylic acid, tartaric acid, particularly (+)-L-tartaric acid, fumaric acid, galactaric acid, citric acid, D-glucuronic acid, lactobionic acid, in anhydrous or a hydrated from thereof, particularly a mono- or di-hydrate.

9. The formulation according to claim 7 or 8, wherein said acid is citric acid, or a hydrate thereof, or fumaric acid, or a hydrate thereof.

10. The formulation according to any one of claims 3 to 9, wherein said pharmaceutically-acceptable excipient is a filler (d).

11. The formulation according to any one of claim 10, wherein said filler (d) is a saccharide-containing filler.

12. The formulation according to claim 11, wherein said saccharide-containing filler is selected from the group comprising, preferably consisting of, lactose, maltodextrin, sucrose, and mannitol.

13. The formulation according to claim 12, wherein said saccharide-containing filler is mannitol.

14. The formulation according to any one of claims 1 to 13, wherein the ratio component a) : component b) is 0.1 : 10, preferably 1 : 5, more preferably 1 : 2, by weight.

15. The formulation according to any one of claims 1 to 14, wherein the ratio component a) : component b) is 1 : 1, by weight.

16. The formulation according to any one of claims 1 to 15, which is in the form of a tablet, a pellet or a powder.

17. The tablet according to claim 16, wherein said 1-[4-chloroanilino]-4-[4-pyridylmethyl]phthalazine, or pharmaceutically acceptable salt thereof, is present in an amount of around 20 to 55%, preferably around 25 to 30%, more preferably 26.8%, by weight of the total weight of said tablet.

18. The tablet according to claim 16 or 17, wherein said tablet is coated.

19. The coated tablet according to any one of claims 16 to 18, wherein said tablet is film-coated.

20. The film-coated tablet according to claim 19, wherein said tablet is a sugar-coated.

21. The powder according to claim 16, which is present in a capsule, particularly a hard gelatin capsule.

22. The capsule according to claim 21, which is coated, preferably film-coated.

23. The powder according to claim 16, which is present in a single dose container, particularly a sachet, more particularly a stick pack.

24. A method of preparing a solid pharmaceutical formulation of a homogeneous dispersion of a) a pharmaceutically acceptable salt of 1-[4-chloroanilino]-4-[4-pyridylmethyl]phthalazine, and b) a pharmaceutically acceptable carboxylic acid having a pKa value of equal to or less than 3.2, as coherent phase, said method comprising the steps of :
i) blending a mixture of said 1-[4-chloroanilino]-4-[4-pyridylmethyl]phthalazine, or a pharmaceutically acceptable salt thereof, and said pharmaceutically acceptable carboxylic acid having a pKa value of equal to or less than 3.2, thereby forming a blended mixture ;
ii) extruding or melting, preferably melt-extruding, and optionally spheronising, said blended mixture, thereby forming an extrudate or melt, particularly in a pellet form ;
iii) obtaining an appropriate particle size distribution of said extrudate or melt, for example by milling or sieving, thereby providing a extrudate or melt of appropriate particle size, which is the above-mentioned solid pharmaceutical formulation.

25. The method according to claim 24, which further comprises the step of :
iv) blending said extrudate of appropriate particle size with at least one pharmaceutically acceptable excipient, and optionally forming, particularly by direct compression, a tablet.

26. The method according to claim 24 or 25, which further comprises the step of :
v) placing said extrudate into a capsule, preferable a hard gelatine capsule.

27. The method according to claim 25 or 26, which further comprises the step of :
vi) placing said extrudate into a single dose container, preferably a sachet.

28. A solid pharmaceutical formulation obtainable by the method according to any one of claims 24 to 27.

29. A solid pharmaceutical formulation according to any of claims 1 to 28, for use as a medicament.

30. Use of a solid pharmaceutical formulation according to any of claims 1 to 28, for the manufacture of a medicament for the treatment of a proliferative disease, particularly cancer.

31. Use of a solid pharmaceutical formulation according to any of claims 1 to 28, for the manufacture of a medicament for the treatment of a proliferative disease, particularly cancer, in combination with a chemotherapeutic agent.

32. A method of treating a proliferative disease, particularly cancer, said method comprising administering a therapeutically effective amount of the solid pharmaceutical formulation according to any of claims 1 to 28, to a patient in need thereof.

33. A method of treating a proliferative disease, particularly cancer, said method comprising administering a therapeutically effective amount of the dispersed solid pharmaceutical formulation according to any of claims 1 to 28, and a chemotherapeutic agent to a patient in need thereof.
